# EUROPEAN PATENT SPECIFICATION

(11) **EP 2 468 222 B1**
(45) Date of publication and mention of the grant of the patent: **27.04.2016**
(21) Application number: 11195452.5
(22) Date of filing: 22.12.2011
(51) Int. Cl.: A61F 7/02, A61F 7/10, A61F 13/15, B65H 45/22, F25D 5/02

(54) **Device for the application of cold**
Vorrichtung zur Kälteanwendung
Dispositif pour l'application de froid

(30) Priority: 22.12.2010 IT RM20100684
(43) Date of publication of application: 27.06.2012
(73) Proprietor: MIC Medical S.r.l, Roma (IT)
(72) Inventor: Matricardi, Pietro, 00189 Roma (IT); Di Meo, Chiara, 00189 Roma (IT); De Marco, Franco, 00189 Roma (IT); Ciolfi, Lucio, 00189 Roma (IT)
(74) Representative: Bergadano, Mirko

(56) References cited:
- US-A- 3 149 943
- US-A- 3 542 032
- US-A- 4 967 573
- US-B1- 6 440 160

## Description

The present invention relates to a device for the application of cold.

In particular, the present invention finds a preferred and advantageous use in the treatment of traumas in which an instantaneous application of cold on the traumatized part is necessary.

For several years there have existed on the market devices that are able to provide instantaneous cold through triggering of endothermic solubilization. For the most part, these devices are constituted by a plastic bag containing both a chemical substance in granular form and a bladder once again made of plastic and filled with water. Once the bladder is burst by a pressure from outside, the chemical substance solubilizes in the water that comes out of the bladder in an endothermic way, with consequent cooling of the solution formed within the bag.

Said devices are particularly convenient for enabling a prompt therapeutic intervention by means of application of cold.

A disadvantage regarding this type of devices lies in the fact that they are necessarily disposable devices, with the obvious disadvantages, in economic, practical, and environmental terms, that this involves.

A further disadvantage of this type of devices regards the fact that the bag cannot be left resting on the part to be treated, but has to be constantly held in position in order to prevent it from sliding off. In fact, the liquid consistency inside the bag does not allow the latter to take on a shape around the traumatized part and remain there stably.

Devices alternative to the ones described above have also for some time been present on the market and basically consist of a gel housed in a plastic bag, which, when kept in a low-temperature environment is able to preserve the cold accumulated without freezing.

The above devices are used following upon preventive cooling thereof and present the major advantage of being usable many times. In fact, once the gel has warmed up, reaching, for example, room temperature, it is possible to cool it again by keeping it for an appropriate length of time in a low-temperature environment. Another advantage of this second type of devices lies precisely in the fact that their gel-like consistency enables the bag to be modelled around the traumatized part with a greater convenience seeing that it does not need to be held in position from outside, and with a greater effectiveness of application of cold. In fact, being able to model the bag around the traumatized part necessarily optimises the surface of contact between the bag and the traumatized part itself.

There has thus been felt the need to have available a device for application of cold, the technical characteristics of which are able to guarantee an instantaneous formation of a cold gel in such a way as to combine the advantages of the two types of devices referred to above. US 5 534 020 is considered to be the closest prior art for claim 1 and discloses a cooling pack with the endothermic composition, the gelling agent and the gel formation favouring agent in one compartment and the water in the other compartment.

The subject of the present invention is a device for the application of cold, the basic characteristics of which are specified in Claim 1, and the preferred and/or auxiliary characteristics of which are specified in Claims 2-16.

For a better understanding of the invention some embodiments are provided hereinafter purely by way of illustrative and non-limiting example, with the aid of the annexed drawings, wherein:
- Figure 1 is a plot of the temperature as a function of time of the gel formed in the device forming the subject of the present invention; and
- Figure 2 is a schematic illustration of a preferred embodiment of the device forming the subject of the present invention.

### Example 1

A solid composition was prepared, made up of 150g of urea and 0.8g of soda both in pellet form and an aqueous solution made up of 200ml of water, 2.4g of polyacrylic acid, and 25ml of ethylene glycol. The solid composition and the aqueous solution were mixed at room temperature in a 300-ml beaker to obtain an instantaneous formation of a cold gel.

The polyacrylic acid used is marketed under the trade name CARBOPOL® 940.

It has been experimentally found that polyacrylic acid, like any other gelling substance used, must be kept in the aqueous solution and not in the solid composition. In this way, in fact, the instantaneousness of the formation of gel can be guaranteed at the moment of mixing. In the case where, instead, the gelling substance were kept in the solid composition, at the moment of mixing the instantaneous gelling sought would not take place.

The instantaneousness of the formation of cold gel is the purpose of the present invention, in so far as only in this way is it possible to achieve the advantages sought, such as the convenience and effectiveness of the application of cold on the traumatized part.

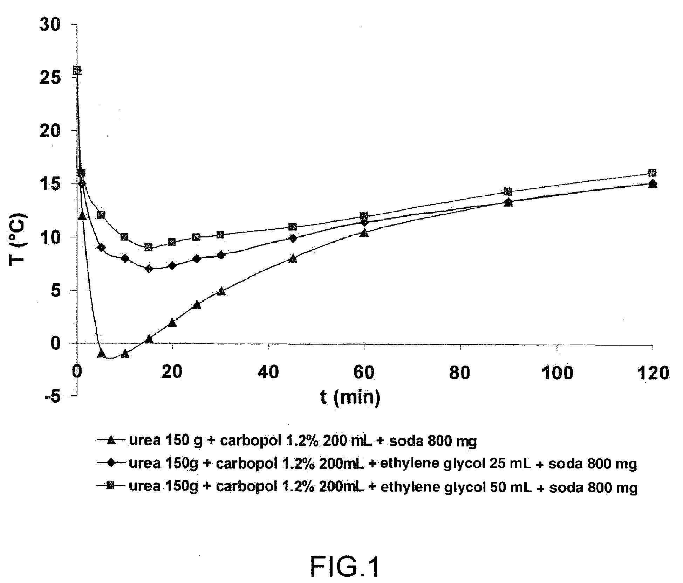

As emerges from the graph in Figure 1, after approximately 15 minutes from the moment of mixing, the gel formed has a temperature of approximately 7°C, reaching 10°C after one hour.

The gel formed, after returning to room temperature, was subsequently kept at a temperature of -10°C for some hours without evidence of freezing, and was thus reusable for a therapeutic intervention by application of cold. It was found that freezing of the gel takes place after it has been kept at a temperature of -16°C.

### Example 2

Example 1 was repeated with the sole difference that the amount of ethylene glycol used in the aqueous solution was 50ml.

In this case, the minimum temperature reached was slightly higher than the one deriving from Example 1, whilst the curve of return to room temperature was practically superimposable on the plot illustrated in Example 1.

The gel formed was kept at a temperature of -16°C without evidence of freezing.

It should be, however, pointed out, in any case, that the possible cooling at such low temperatures, as -16°C, cannot constitute a limitation in so far as said temperatures cannot be functional for use of the device.

### Example 3

Example 1 was repeated with the sole difference that ethylene glycol was not used.

In this case, as illustrated in Figure 1, the minimum temperature reached was clearly lower than the one deriving from Example 1, whilst the curve of return to room temperature was practically superimposable on the plot illustrated in Example 1.

Furthermore, it was found that the gel, once brought to room temperature, can be preserved down to a temperature of -5°C for some hours without any evidence of freezing even though the antifreeze compound is not present in the gel.

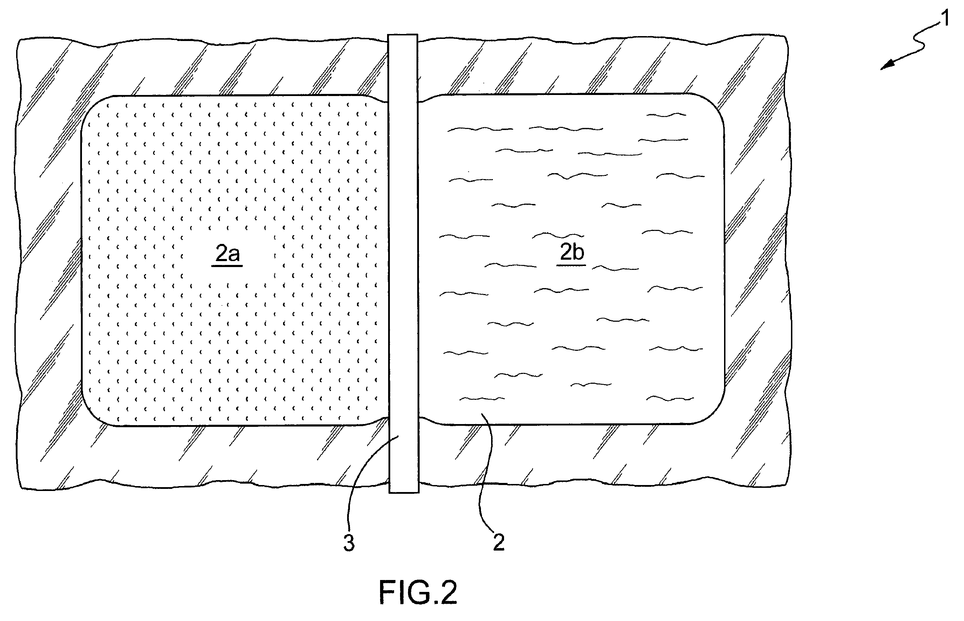

Designated as a whole by 1 in Figure 2 is a device for instantaneous production of cold gel according to the present invention.

The device 1 is made up of a closed plastic bag 2 and a removable separation element 3 which divides the bag 2 into two compartments 2a and 2b hermetically separated from one another. The removable separation element 3 is formed by two sticks shaped in such a way as to slot longitudinally into one another. In this way, the element 3 is arranged so as to engage the opposite walls of the bag 2 forcing them against one another in a position corresponding to slotting of the two sticks and dividing the bag 2 itself into a first compartment 2a and a second compartment 2b.

Housed in the first compartment 2a is the solid composition as described above, whilst housed in the second compartment 2b is the aqueous solution as defined above.

Once the removable separation element 3 is removed by means of an action of unslotting of the two sticks, the contents of the two compartments 2a and 2b are mixed causing the endothermic reaction of solubilization and the instantaneous simultaneous formation of gel. At this point, the bag 2 will be filled with a cold gel and may be applied on the traumatized part that needs the application of cold.

The division of the bag as described above guarantees a fast and effective mixing, which proves necessary for instantaneous production of a cold gel. In fact, the removal of the element 3 immediately brings the bag back into the state where it has a single volume, thus guaranteeing a high and effective mixing of the contents of the two compartments 2a and 2b. Furthermore, the separation element 3 is completely external to the bag with the advantage of not leaving therein bodies foreign to the gel that has formed. Finally, the structure of the device described above guarantees a safe storage in so far as the accidental removal of the separation element 3 is practically prevented. Instead, devices having a bladder of water inside the bag are readily subject to accidental activation during storage since the bladder itself can burst as a result of any external pressure.

To sum up, the present invention guarantees an instantaneous formation of a cold gel to be applied on a traumatized part, with the advantages of positioning the bag in a stable way on the traumatized part and, at the same time, optimizing the surface of contact with a greater effectiveness in the application of cold. Instead, the devices of the known art, in which the cold substance is liquid, do not manage to remain in a stable position on the traumatized part of the body and are unable to model themselves effectively to be able to guarantee a surface of contact as large as possible for the transmission of cold.

Furthermore, once the gel formed is heated, reaching, for example, room temperature, it is possible to cool it again, keeping it for the necessary time in a low-temperature environment without any danger of it freezing.

In this way, the device does not have to be thrown away, as instead occurs for the disposable devices present today on the market, but may continue to be used as in the case of gels of the known art, which are able to preserve the cold.

## Claims

1. A device (1) for the application of cold comprising a bag (2) divided by removable separation means (3) into two compartments (2a, 2b) hermetically separated from one another; said device being **characterized in that** said first compartment (2a) houses a solid composition comprising a compound designed to solubilize in an endothermic way in water and a substance designed to favour formation of a gel by modulation of pH and/or of ionic force of an aqueous solution, and **in that** said second compartment (2b) houses an aqueous solution comprising water **characterized in that** a gelling substance is also located in the second compartment.

2. The device for the application of cold according to Claim 1, **characterized in that** said aqueous solution comprises an antifreeze compound.

3. The device for the application of cold according to Claim 1 or Claim 2, **characterized in that** said compound solubilizes in water in an endothermic way without releasing ions.

4. The device for the application of cold according to Claim 3, **characterized in that** said compound designed to solubilize in an endothermic way in water is urea.

5. The device for the application of cold according to any one of the preceding claims, **characterized in that** said compound designed to solubilize in an endothermic way in water is used in the form of pellets.

6. The device for the application of cold according to any one of the preceding claims, **characterized in that** said gelling substance is polyacrylic acid.

7. The device for the application of cold according to any one of the preceding claims, **characterized in that** said gelling substance is present in said aqueous solution in an amount comprised between 0.2 and 5% w/v.

8. The device for the application of cold according to any one of the preceding claims, **characterized in that** said substance designed to favour formation of a gel is a basic substance.

9. The device for the application of cold according to Claim 8, **characterized in that** said basic substance is comprised in the group consisting of hydroxides and amines.

10. The device for the application of cold according to Claim 9, **characterized in that** said basic substance is present in said solid composition in an amount such as to bestow upon the gel formed a value of pH comprised between 6 and 10.

11. The device for the application of cold according to any one of Claims 8 to 10, **characterized in that** said basic substance is present in said solid composition according to a weight ratio with respect to said gelling substance comprised between 0.2 and 1.

12. The device for the application of cold according to Claim 2, **characterized in that** said antifreeze compound is ethylene or propylene glycol.

13. The device for the application of cold according to Claim 2, **characterized in that** said antifreeze compound is present in said aqueous solution in an amount comprised between 5 and 50% v/v.

14. The device for the application of cold according to Claim 2, **characterized in that** said antifreeze compound is present in said aqueous solution in an amount comprised between 10 and 20% v/v.

15. The device for the application of cold according to any one of the preceding claims, **characterized in that** said removable separation means (3) engage from outside two opposite walls of said bag forcing them on one another so as to create the two compartments (2a, 2b) hermetically separated from one another.

16. The device for the application of cold according to Claim 15, **characterized in that** said removable separation element (3) is constituted by two sticks shaped in such a way as to slot longitudinally into one another.

## Patentansprüche

1. Einrichtung (1) für die Anwendung von Kälte, aufweisend eine Tasche (2), welche durch entfernbare Trennmittel (3) in zwei Kammern (2a, 2b) geteilt ist, welche hermetisch voneinander getrennt sind; wobei die Einrichtung **dadurch gekennzeichnet ist, dass** die erste Kammer (2) eine feste Zusammensetzung aufnimmt, welche eine erste Verbindung enthält, welche ausgebildet ist, um sich in einer endothermen Weise in Wasser aufzulösen, und eine Substanz, welche ausgebildet ist, eine Formation eine Gels durch eine Modulation von pH und/oder einer Ionenkraft einer wässrigen Lösung zu begünstigen, und dadurch, dass die zweite Kammer (2b) eine wässrige Lösung aufnimmt, welche Wasser enthält, **dadurch gekennzeichnet, dass** eine Gelbildungssubstanz auch in der zweiten Kammer angeordnet ist.

2. Einrichtung für die Anwendung von Kälte nach Anspruch 1, **dadurch gekennzeichnet, dass** die wässrige Lösung eine Frostschutzmittelverbindung beinhaltet.

3. Einrichtung für die Anwendung von Kälte nach Anspruch 1 oder Anspruch 2, **dadurch gekennzeichnet, dass** die Verbindung sich in Wasser in einer endothermen Weise ohne die Freisetzung von Ionen löst.

4. Einrichtung für die Anwendung von Kälte gemäß Anspruch 3, **dadurch gekennzeichnet, dass** die Verbindung, welche ausgebildet ist, um sich in einer endothermen Weise in Wasser zu lösen, Harnstoff ist.

5. Einrichtung für die Anwendung von Kälte gemäß irgendeinem der vorhergehenden Ansprüche, **dadurch gekennzeichnet, dass** die Verbindung, welche ausgebildet ist, um sich in einer endothermen Weise in Wasser zu lösen, in der Form von Pellets verwendet wird.

6. Einrichtung für die Anwendung von Kälte nach irgendeinem der vorhergehenden Ansprüche, **dadurch gekennzeichnet, dass** die Gelbildungssubstanz Polyacrylsäure ist.

7. Einrichtung für die Anwendung von Kälte gemäß irgendeinem der vorhergehenden Ansprüche, **dadurch gekennzeichnet, dass** die Gelbildungssubstanz in der wässrigen Lösung in einem Betrag vorhanden ist, welcher zwischen 0,2 und 5 % m/V (Massenkonzentration) beinhaltet ist.

8. Einrichtung für die Anwendung von Kälte gemäß irgendeinem der vorhergehenden Ansprüche, **dadurch gekennzeichnet, dass** die Substanz, welche für die Begünstigung der Gelbildung ausgebildet ist, eine basische Substanz ist.

9. Einrichtung für die Anwendung von Kälte gemäß Anspruch 8, **dadurch gekennzeichnet, dass** die basische Substanz aus der Gruppe zusammengesetzt ist, welche aus Hydroxiden und Aminen zusammengesetzt ist.

10. Einrichtung für die Anwendung von Kälte nach Anspruch 9, **dadurch gekennzeichnet, dass** die basische Substanz in der festen Zusammensetzung in einem Betrag vorhanden ist, um dem Gel, welches ausgebildet wird, einen pH-Wert zwischen 6 und 10 zu verleihen.

11. Einrichtung für die Anwendung von Kälte gemäß irgendeinem der Ansprüche 8 bis 10, **dadurch gekennzeichnet, dass** die basische Substanz in der festen Zusammensetzung gemäß einem Gewichtsverhältnis zwischen 0,2 und 1 mit Bezug auf die Gelbildungssubstanz vorhanden ist.

12. Einrichtung für die Anwendung von Kälte nach Anspruch 2, **dadurch gekennzeichnet, dass** die Frostschutzmittelverbindung Ethylen oder Propylenglykol ist.

13. Einrichtung für die Anwendung von Kälte gemäß Anspruch 2, **dadurch gekennzeichnet, dass** die Frostschutzmittelverbindung in der wässrigen Lösung in einem Betrag zwischen 5 und 50 % V/V (Volumenanteil) vorhanden ist.

14. Einrichtung für die Anwendung von Kälte gemäß Anspruch 2, **dadurch gekennzeichnet, dass** die Frostschutzmittelverbindung in der wässrigen Lösung in einem Betrag zwischen 10 und 20 % V/V (Volumenanteil) vorhanden ist.

15. Einrichtung für die Anwendung von Kälte gemäß irgendeinem der vorhergehenden Ansprüche, **dadurch gekennzeichnet, dass** die entfernbaren Trennmittel (3) von der Außenseite zwei gegenüberliegende Wände der Tasche ergreifen, und diese aneinander zwingen, um die zwei Kammern (2a, 2b) zu bilden, welche hermetisch voneinander getrennt sind.

16. Einrichtung für die Anwendung von Kälte gemäß Anspruch 15, **dadurch gekennzeichnet, dass** die entfernbaren Trennmittel (3) durch zwei Stöcke ausgebildet sind, welche in solch einer Weise geformt sind, um sich der Länge nach ineinander einzuschlitzen.

## Revendications

1. Dispositif (1) pour appliquer du froid comprenant une poche (2) divisée par des moyens de séparation (3) déplaçables en deux compartiments (2a, 2b) séparés hermétiquement l'un de l'autre ; ledit dispositif étant **caractérisé en ce que** ledit premier compartiment (2a) contient une composition solide comportant un composé qui se dissout dans l'eau de façon endothermique et une substance agencée pour favoriser la formation d'un gel suite à la modulation du pH et/ou de la force ionique d'une solution aqueuse, et **en ce que** ledit second compartiment (2b) contient une solution aqueuse comportant de l'eau, **caractérisée en ce qu'**une substance gélifiante est également contenue dans le second compartiment.

2. Dispositif pour appliquer du froid, selon la revendication 1, **caractérisé en ce que** ladite solution aqueuse comporte un composé antigel.

3. Dispositif pour appliquer du froid, selon la revendication 1 ou la revendication 2, **caractérisé en ce que** ledit composé se dissout dans l'eau de façon endothermique sans libérer d'ions.

4. Dispositif pour appliquer du froid, selon la revendication 3, **caractérisé en ce que** ledit composé qui se dissout dans l'eau de façon endothermique est de l'urée.

5. Dispositif pour appliquer du froid, selon l'une quelconque des revendications précédentes, **caractérisé en ce que** ledit composé qui se dissout dans l'eau de façon endothermique est utilisé sous la forme de pellets.

6. Dispositif pour appliquer du froid, selon l'une quelconque des revendications précédentes, **caractérisé en ce que** ladite substance gélifiante est de l'acide polyacrylique.

7. Dispositif pour appliquer du froid, selon l'une quelconque des revendications précédentes, **caractérisé en ce que** ladite substance gélifiante est présente dans ladite solution aqueuse dans une proportion comprise entre 0,2 et 5% p/v.

8. Dispositif pour appliquer du froid, selon l'une quelconque des revendications précédentes, **caractérisé en ce que** ladite substance agencée pour favoriser la formation d'un gel est une substance basique.

9. Dispositif pour appliquer du froid, selon la revendication 8, **caractérisé en ce que** ladite substance basique fait partie du groupe consistant en hydroxydes et en amines.

10. Dispositif pour appliquer du froid, selon la revendication 9, **caractérisé en ce que** ladite substance basique est présente dans ladite composition solide selon une proportion telle que le pH du gel produit a une valeur comprise entre 6 et 10.

11. Dispositif pour appliquer du froid, selon l'une quelconque des revendications 8 à 10, **caractérisé en ce que** ladite substance basique est présente dans ladite composition solide selon la proportion en poids par rapport à ladite substance gélifiante comprise entre 0.2 et 1.

12. Dispositif pour appliquer du froid, selon la revendication 2, **caractérisé en ce que** ledit composé antigel est de l'éthylène ou du propylène glycol.

13. Dispositif pour appliquer du froid, selon la revendication 2, **caractérisé en ce que** ledit composé antigel est présent dans ladite solution aqueuse dans une proportion comprise entre 5 et 50% v/v.

14. Dispositif pour appliquer du froid, selon la revendication 2, **caractérisé en ce que** ledit composé antigel est présent dans ladite solution aqueuse dans une proportion comprise entre 10 et 20% v/v.

15. Dispositif pour appliquer du froid, selon l'une quelconque des revendications précédentes, **caractérisé en ce que** lesdits moyens de séparation (3) déplaçables appuient sur les parois opposées de ladite poche de manière à créer les deux compartiments (2a, 2b) séparés hermétiquement l'une de l'autre.

16. Dispositif pour appliquer du froid, selon la revendication 15, **caractérisé en ce que** ledit élément de séparation (3) déplaçable est constitué de deux tiges configurées de telle manière qu'elles se fixent longitudinalement l'une contre l'autre.
